# EUROPÄISCHE PATENTSCHRIFT

(11) **EP 0 981 325 B1**
(45) Veröffentlichungstag und Bekanntmachung des Hinweises auf die Patenterteilung: **13.10.2004**
(21) Anmeldenummer: 98925520.3
(22) Anmeldetag: 02.05.1998
(51) Int. Cl.: A61K 7/48

(54) **HAUTPFLEGEMITTEL**
SKIN CARE PRODUCTS
PRODUITS POUR LE SOIN DE LA PEAU

(30) Priorität: 12.05.1997 DE 19719856
(43) Veröffentlichungstag der Anmeldung: 01.03.2000
(73) Patentinhaber: Henkel KGaA, 40589 Düsseldorf (DE)
(72) Erfinder: MUNK, Gabriele, D-40593 Düsseldorf (DE); WALDMANN-LAUE, Marianne, D-40789 Monheim (DE); BORDAT, Pascal, 31130 Flourens (FR); JASSOY, Claudia, D-40235 Düsseldorf (DE)
(86) Internationale Anmeldenummer: PCT/EP1998/002596
(87) Internationale Veröffentlichungsnummer: WO 1998/051275

(56) Entgegenhaltungen:
- EP-A- 0 769 291
- WO-A-92/09636
- WO-A-97/02012
- DE-A- 3 443 985
- DATABASE WPI Section Ch, Week 9247 Derwent Publications Ltd., London, GB; Class A96, AN 92-387690 XP002077163 & JP 04 288017 A (PIAS KK) , 13. Oktober 1992

## Beschreibung

Die Erfindung betrifft kosmetische und dermatologische Zubereitungen zur Pflege empfindlicher oder gereizter Haut, die aufgrund einer besonders ausgewählten Wirkstoffkombination eine hautberuhigende und die entzündlichen Hautzustände bei Akne hemmende Wirkung aufweisen.

Die kosmetische Pflege der empfindlichen Haut hat in jüngster Zeit an Bedeutung gewonnen, da viele Menschen ihre Haut als empfindlich einstufen, andere durch häufige Einwirkung von Sonnenstrahlung oder von hautreizenden Agenzien ihre Haut belasten, oder an allergischen und entzündlichen Zuständen der Haut erkrankt sind. Insbesondere die Akne ist als ein Hautzustand bekannt, der auch in leichten Fällen schon als sehr störend empfunden wird.

Es hat daher nicht an Versuchen gefehlt, durch kosmetische und dermatologische Hautbehandlungsmittel solchen Hautzuständen vorzubeugen oder diese zu heilen.

Chitosan, ein partiell deacetyliertes Chitin, also ein freies Aminogruppen-tragendes Biopolymer, ist als kosmetischer Rohstoff mit einer günstigen Wirkung auf die Weichheit und Elastizität sowie auf den Feuchtigkeitsgehalt der Haut und als Polymeres mit einer gewissen antimikrobiellen Wirkung bekannt.

Auch Zink-Salze sind als antimikrobielle und antimykotische Komponenten in dermatologischen Zubereitungen, z.B. in DE 34 43 985 schon beschrieben worden.

Die WO-A-9209636 offenbart ein Verfahren zum Schutz der Haut vor einem Kontakt mit einem allergenen Agens bei dem eine Zusammensetzung mit einem Gehalt an 6 Gew.% Chitosan-lactat und 0,11 Gew.% Zinkacetat verwendet wird.

Es wurde nun aber überraschend gefunden, daß Chitosane in Zusammensetzungen mit gelösten Zink-Salzen eine besonders günstige kosmetische und dermatologische Wirkung auf gereizte oder entzündete Haut ausüben und daß sich diese Stoffe nicht nur in ihren bekannten Wirkungen ergänzen, sonderen darüber hinaus eine erhöhte, hautberuhigende und heilungsfördernde Wirkung zeigen.

Gegenstand der Erfindung sind daher Hautpflegemittel mit hautberuhigender Wirkung, die einen Gehalt von 0,01 bis 1,0 Gew.-% Chitosan in Form eines gelösten Salzes und 10 bis 500 ppm Zink in Form eines gelösten Salzes in einem wäßrigen Träger enthalten. Als Hautpflegemittel im Sinne der Erfindung sind dabei alle zur Anwendung auf der Haut, der Kopfhaut und den Schleimhäuten anwendbaren Zubereitungen zu verstehen, die wenigstens sowiel Wasser enthalten, daß wenigstens 0,01 Gew.-% des Chitosans und wenigstens 10 ppm Zink-Ionen in gelöster Form darin vorliegen können. Solche Zubereitungen können wäßrige Lösungen, Gele, Emulsionen, Cremes, Stiftpräparate, Schaumaerosole oder Sprays sein.

Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 19 503 465 A1 beschrieben.

Besonders gut geeignete Chitosane weisen einen Deacetylierungsgrad von wenigstens 80 % und ein Molekulargewicht von 5·10⁵ bis 5·10⁶ (g/mol) auf.

Zur Herstellung erfindungsgemäßer Zubereitungen muß das Chitosan in die Salzform überführt werden. Dies kann durch Auflösen in verdünnten wäßrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren. Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, Benzoesäure oder Salycilsäure. Bevorzugt werden niedermolekulare Hydroxycarbonsäuren wie z.B. Glycolsäure oder Milchsäure verwendet.

Auch das Zink liegt in Form eines gelösten Salzes vor. Als Zinksalze eignen sich prinzipiell alle wasserlöslichen Salze, sowohl von Mineralsäuren als auch von organischen Säuren. Geeignete Zinksalze sind also z.B. das Chlorid, das Sulfat, das Acetat, das Glycolat, das Lactat, das Citrat, das Gluconat, das Salicylat, das Glycinat, das Aspartat oder das Pantothenat. Geeignet sind aber auch Salze von Organosulfonsäuren und Organophosphorsäuren wie z.B. das Zink-Phenolsulfonat oder das Zink-Phytat.

Bevorzugt sind solche erfindungsgemäße Hautpflegemittel, die das Chitosan und das Zink in Form von Salzen von Hydroxycarbonsäuren oder Polyhydroxycarbonsäuren enthalten. In einer besonders bevorzugten Ausführung ist das Chitosan in Form eines Glycolats und das Zink in Form von Zink-Gluconat enthalten.

Als wasserhaltiger Träger kann im einfachsten Fall Wasser oder ein wäßriger Alkohol dienen. Bevorzugt enthält der wäßrige Träger aber weitere Hilfsmittel, die dazu geeignet sind, die erfindungsgemäßen Hautpflegemittel dem jeweiligen Anwendungszweck anzupassen.

So sind z.B. Tenside enthalten, um die Benetzungseigenschaften zu erhöhen, um wasserunlösliche Hilfsmittel zu emulgiern oder zu solubilisieren oder um die Produkte verschäumbar zu machen. Wasserlösliche Verdickungsmittel können dazu dienen, den Produkten mehr "Körper" zu verleihen oder ihre Haftung auf der Haut zu verbessern, emulgierte Öle können enthalten sein, um den Produkten auch eine hautglättende und rückfettende Wirkung zu verleihen.

Bevorzugt enthält der wäßrige Träger Tenside, wasserlösliche Verdickungsmittel, emulgierte Ölkomponenten oder Gemische davon.

Geeignete Tenside sind vor allem die gut wasserlöslichen anionischen, zwitterionischen, ampholytischen oder nichtionischen oberflächenaktiven Stoffe. Diese zeichnen sich bevorzugt durch eine lineare Alkyl- oder Acylgruppe mit 10 bis 16 C-Atomen und eine endständig daran befindliche wasserlöslichmachende Gruppe, z.B. eine Carboxylat-, Sulfat- oder Polyoxyethyl- oder Polyolgruppe aus. Bevorzugt geeignet sind die nichtionischen Tenside, z.B. die Fettalkoholpolyglycolether, die Fettsäuremonoglycerid-Oxethylate, die Sorbitan-Fettsäureester-Oxethylate, die Alkylglycoside und die Methylglucosid-Fettsäureester-Oxethylate. Neben diesen Emulgatoren können lipophile Coemulgatoren, z.B. C₁₂-C₁₈-Fettalkohole und C₁₂-C₁₈-Fettsäurepartialglyceride enthalten sein.

Als wasserlösliche Verdickungsmittel eignen sich natürliche, biotechnologische und synthetische, bevorzugt nichtionische Hydrocolloide. Natürliche Verdickungsmittel sind z.B. Pflanzengumme, wie z.B. Agar Agar, Stärke, Gelatine, Guar und deren Derivate sowie Cellulosederivate, biotechnologisch erzeugte Hydrocolloide sind z.B. Xanthan- und Succinoglycan, synthetische wasserlösliche Verdickungsmittel sind z.B. Polyvinylalkohol und Polyvinylpyrrolidon.

Als emulgierte Ölkomponenten können alle zur Herstellung kosmetischer Emulsionen oder Cremes geeigneten Öle, Fette und Wachse mineralischer, tierischer und pflanzlicher Herkunft enthalten sein. Neben den genannten Trägerkomponenten können alle üblichen Hilfsmittel für die Herstellung kosmetischer Hautpflegemittel oder dermatologischer Extema enthalten sein. Solche weiteren Hilfsmittel sind z.B. niedere Alkohole, z.B. Ethanol oder Isopropanol, Glycole und Polyole wie z.B. Ethylenglycol, Propylenglycol, Dipropylenglycol, Glycerin, Di- bzw. Polyglycerin, Sorbit, Harnstoff, Glucose, Salze, z.B. Mangnesiumsulfat, Antioxidantien, Komplexbildner, Konservierungsmittel, Duftstoffe, Farbstoffe, Trübungsmittel und kosmetische Wirkstoffe, z.B. Lichtschutzfiltersubstanzen, heilungsfördernde Stoffe wie z.B. Panthenol oder Pantothensäure (Salze), Vitamine oder Pflanzenextrakte.

Besonders bevorzugt sind solche erfindungsgemäßen Hautpflegemittel, die Panthenol oder Salze der Pantothensäure, z.B. Ca-Pantothenat, in einer Menge von 0,1 - 1 Gew.-% enthalten.

Wie weiter oben ausgeführt, können die erfindungsgemäßen Hautpflegemittel sehr verschiedene Anwendungsformen aufweisen. Bevorzugt liegen sie jedoch als Gesichtswasser, als Pflegegel oder als Öl-in-Wasser-Emulsion oder Creme vor. Solche Zubereitungen eignen sich insbesondere auch zur Anwendung nach dem Sonnenbad, also als After-Sun-Präparate, um der gereizten und geröteten Haut wieder die erforderliche Feuchtigkeit zuzuführen und eine beschleunigte Beruhigung der gereizten Haut sowie eine rasche Heilung zu bewirken. Eine weitere Indikation für die erfindungsgemäßen Hautpflegemittel ist die Reinigung und die Pflege der von Akne befallenen Haut. Hier kommt die hautberuhigende Wirkung der erfindungsgemäßen Kombination besonders gut zur Geltung.

Ein besonders bevorzugter Gegenstand der vorliegenden Erfindung ist daher die Verwendung einer Kombination von Chitosan und Zink in Form von gelösten Salzen zur

Herstellung einer wäßrigen, topischen Zubereitung zur Beruhigung gereizter Haut und zur Vorbeugung vor oder Behandlung von Akne.

Solche Zubereitungen eignen sich insbesondere als kosmetische Hautbehandlungsmittel zur Ergänzung der bekannten, rigoroseren Akne-Therapie.

Die folgenden Beispiele sollen den Erfindungsgegenstand näher erläutern:

### Beispiele

### 1. Anwendungsbeispiele

| **1.1 Gesichtswasser** | **1.1.1** | **1.1.2** | **1.1.3** |
|---|---|---|---|
| Pluronic®L 64 (1) | 3 | 4 | 5 |
| d-Panthenol | 0,25 | 0,25 | 0,25 |
| Dipropylenglycol | 10 | 10 | 10 |
| Emulgator TD9/PEG40HCO (2) | 0,5 | 0,5 | 0,5 |
| Duftstoff | 0,2 | 0,2 | 0,2 |
| Zn-Gluconat | 0,05 | 0,07 | 0,10 |
| Hydagen®CMF (3) | 3,5 | 6,0 | 9,5 |
| Wasser (NaOH bis pH = 5 ± 0,2) | ad 100 | ad 100 | ad 100 |

### Herstellung:

Die Rohstoffe werden nacheinander in das vorgelegte, auf 40°C erwärmte Wasser eingerührt. Der Emulgator wird vorher mit Dipropylenglycol und dem Duftstoff vorgemischt und dann zugesetzt. Zuletzt wird die Chitosan-Lösung (Hydagen®CMF) in die Zubereitung eingerührt und der pH-Wert mit Natronlauge auf 5 ± 0,2 eingestellt.

| **1.2 Hydrogele** | **1.2.1** | **1.2.2** |
|---|---|---|
| Pluronic®L64 (1) | 3,0 | 3,0 |
| Methocel®E4M (4) | 0,3 | 0,20 |
| d-Panthenol | 0,25 | 0,25 |
| Dipropylenglycol | 10,0 | 10,0 |
| Emulgator TD9/PEG40HCO (2) | 0,5 | 0,50 |
| Riechstoff | 0,2 | 0,20 |
| Zn-Gluconat | 0,05 | 0,10 |
| Hydagen CMF (3) | 3,5 | 8,0 |
| Wasser (NaOH bis pH = 5 ± 0,2) | ad 100 | ad 100 |

### Herstellung:

Der Emulgator wird mit dem Dipropylenglycol und dem Duftstoff vorgemischt. Die Methylcellulose wird in einem Teil des Wassers vorgelöst. Dann werden diese Vorgemische und die übrigen Rohstoffe dem vorgelegten Wasser unter Rühren zugegeben. Zuletzt wird der pH-Wert auf 5 ± 0,2 durch Natronlauge eingestellt.

| **1.3 Hautemulsion (O/W)** | | |
|---|---|---|
| Emulgade®SE (5) | 8,0 | 8,0 |
| Cutina®MD-A (6) | 1,5 | 1,5 |
| Cetyl-/Stearylalkohol | 1,5 | 1,5 |
| Myritol®318 (7) | 10,0 | 10,0 |
| 2-Ethylhexyl-Stearat | 5,0 | 5,0 |
| Dimethylpolysiloxan (350 at) | 1,0 | 1,0 |
| Controx®KS (8) | 0,05 | 0,05 |
| PHB-Propylester | 0,2 | 0,2 |
| PHB-Methylester | 0,2 | 0,2 |
| 1,2-Propylenglycol | 3,0 | 3,0 |
| Hydagen®CMF (3) | 3,0 | 8,0 |
| Zn-Gluconat | 0,04 | 0,10 |
| Wasser (NaOH bis pH = 5 ± 0,2) | ad 100 | ad 100 |

### Herstellung:

Die Rohstoffe der Fettphase werden gemeinsam auf 80°C erwärmt und gemischt. Das Wasser mit dem 1,2-Propylenglycol und dem PHB-Methylester wird ebenfalls auf 80°C erwärmt und unter Rühren zur Fettphase gegeben. Nach dem Abkühlen wird bei ca. 35°C das Zn-Gluconat im Ansatz gelöst und zum Schluß wird die Hydagenlösung zugesetzt und mit Natronlauge der pH-Wert auf 5 ± 0,2 eingestellt.

In den Beispielen wurden die folgenden Rohstoffe verwendet:

| | | |
|---|---|---|
| (1) Pluronic®L64 | EO-PO-EO-Blockpolymer (EO=40 Gew.-%), OHZ = 39,1 | |
| (2) Emulgator | Trideceth 9 und PEG40-hydrogenated castor oil | |
| (3) Hydagen®CMF | Lösung von Chitosan (ca. 1 Gew.-%) in einer 0,4 %igen | |
| | wäßrigen Glycolsäure-Lösung | |
| (4) Methocel E4M | Methyl-hydroxypropyl-cellulose | |
| (5) Emulgade SE | Gemisch aus: | Glyceryl Stearate |
| | | Ceteareth 20 |
| | | Ceteareth 12 |
| | | Cetearyl Alcohol und |
| | | Cetylpalmitat |
| (6) Cutina MD-A | Glyceryl Stearate | |
| (7) Myritol 318 | Caprylic/Capric-Triglyceride | |
| (8) Controx®KS | Tocopherol und Hydrogenated Tallow | |
| | Glycerides Citrate | |

## Patentansprüche

1. Hautpflegemittel mit hautberuhigender Wirkung, **gekennzeichnet durch** einen Gehalt von
| | |
|---|---|
| 0,01 - 1,0 Gew.-% | Chitosan in Form eines gelösten Salzes und |
| 10 - 500 ppm | Zink in Form eines gelösten Salzes |
in einem wasserhaltigen Träger.

2. Hautpflegemittel nach Anspruch 1, **dadurch gekennzeichnet, daß** der wäßrige Träger Tenside, lösliche Verdickungsmittel, emulgierte Ölkomponenten oder ein Gemisch davon enthält.

3. Hautpflegemittel nach Anspruch 1 oder 2, **dadurch gekennzeichnet, daß** das Chitosan einen Deacetylierungsgrad von wenigstens 80 % und ein Molekulargewicht von 5 · 10⁵ bis 5 · 10⁶ (g/mol) aufweist.

4. Hautpflegemittel nach Anspruch 1 - 3, **dadurch gekennzeichnet, daß** das Zink in Form eines gelösten Salzes einer organischen Säure, insbesondere einer Hydroxycarbonsäure, Polyhydroxycarbonsäure, Organosulfonsäure oder Organophosphorsäure, vorliegt.

5. Hautpflegemittel nach Anspruch 1 bis 4, **dadurch gekennzeichnet, daß** das Chitosan und das Zink in Form von Salzen von Hydroxycarbonsäuren oder Polyhydroxycarbonsäuren enthalten sind.

6. Hautpflegemittel nach Anspruch 1 bis 5, **dadurch gekennzeichnet, daß** das Chitosan in Form eines Glycolats und das Zink in Form von Zinkgluconat enthalten ist.

7. Hautpflegemittel nach einem der Ansprüche 1 bis 6, **dadurch gekennzeichnet, daß** Panthenol oder Salze der Pantothensäure in einer Menge von 0,1 - 1 Gew.-% darin enthalten ist.

8. Hautpflegemittel nach einem der Ansprüche 1 bis 7, **dadurch gekennzeichnet, daß** sie in Form eines Gesichtswassers, eines Pflegegels oder einer Öl-in-Wasser-Emulsion oder Creme vorliegen.

9. Verwendung einer Kombination von Chitosan und Zink in Form gelöster Salze zur Herstellung einer wäßrigen, topischen Zubereitung zur Beruhigung gereizter Haut, zur Vorbeugung vor Akne, zur Behandlung der Akne oder zur Anwendung nach dem Sonnenbad als After-Sun-Präparat.

## Claims

1. Soothing skin-care preparations, **characterized in that** they contain 0.01 to 1.0% by weight of chitosan in the form of a dissolved salt and 10 to 500 ppm of zinc in the form of a dissolved salt in a water-containing carrier.

2. Skin-care preparations as claimed in claim 1, **characterized in that** the aqueous carrier contains surfactants, soluble thickeners, emulsified oil components or a mixture thereof.

3. Skin-care preparations as claimed in claim 1 or 2, **characterized in that** the chitosan has a degree of deacetylation of at least 80% and a molecular weight of 5·10⁵ to 5·10⁶ (g/mol).

4. Skin-care preparations as claimed in claims 1 to 3, **characterized in that** the zinc is present in the form of a dissolved salt of an organic acid, more particularly a hydroxycarboxylic acid, polyhydroxycarboxylic acid, organosulfonic acid or organophosphoric acid.

5. Skin-care preparations as claimed in claims 1 to 4, **characterized in that** the chitosan and the zinc are present in the form of salts of hydroxycarboxylic acids or polyhydroxycarboxylic acids.

6. Skin-care preparations as claimed in claims 1 to 5, **characterized in that** the chitosan is present in the form of a glycolate and the zinc is present in the form of zinc gluconate.

7. Skin-care preparations as claimed in any of claims 1 to 6, **characterized in that** panthenol or salts of pantothenic acid is/are present in a quantity of 0.1 to 1% by weight.

8. Skin-care preparations as claimed in any of claims 1 to 7, **characterized in that** they are present in the form of a face lotion, a care gel or an oil-in-water emulsion or cream.

9. The use of a combination of chitosan and zinc in the form of dissolved salts for the production of a water-based topical preparation for soothing irritated skin, for preventing or treating acne or for application after sunbathing as an after-sun preparation.

## Revendications

1. Produits de soin pour la peau avec un effet calmant pour la peau, **caractérisés par** une teneur de
0,01 - 1,0 % en poids en chitosane sous forme d'un sel dissous et
10 à 500 ppm en zinc sous forme d'un sel dissous
dans un support contenant de l'eau.

2. Produits de soin pour la peau selon la revendication 1, **caractérisés en ce que** le support aqueux contient des agents tensioactifs des épaississants solubles, des composants huileux émulsifiés ou un mélange de ceux-ci.

3. Produits de soin pour la peau selon la revendication 1 ou 2, **caractérisés en ce que** le chitosane présente un degré de désacétylation d'au moins 80% et un poids moléculaire de 5.10⁵ à 5.10⁶ g/mole.

4. Produits de soin pour la peau selon la revendication 1 à 3, **caractérisés en ce que** le zinc se trouve sous forme d'un sel dissous d'un acide organique, en particulier d'un acide hydroxycarboxylique, polyhydroxycarboxylique, organosulfonique ou organophosphorique.

5. Produits de soin pour la peau selon la revendication 1 à 4, **caractérisés en ce que** le chitosane et le zinc sont contenus sous forme de sels d'acides hydroxycarboxyliques ou d'acides polyhydroxycarboxyliques.

6. Produits de soin pour la peau selon la revendication 1 à 5, **caractérisés en ce que** le chitosane est contenu sous forme d'un glycolate et le zinc sous forme d'un gluconate de zinc.

7. Produits de soin pour la peau selon l'une quelconque des revendications 1 à 6, **caractérisés en ce qu'**ils contiennent du panthénol ou des sels de l'acide pantothénique en une quantité de 0,1 à 1% en poids.

8. Produits de soin pour la peau selon l'une quelconque des revendications 1 à 7, **caractérisés en ce qu'**ils se trouvent sous forme d'une lotion pour le visage, d'un gel de soin ou d'une émulsion huile-dans-eau ou crème.

9. Utilisation d'une combinaison de chitosane et de zinc sous forme de sels dissous pour la préparation d'une composition aqueuse topique pour calmer une peau irritée, pour la prophylaxie de l'acné, pour le traitement de l'acné ou pour une utilisation après un bain de soleil comme préparation après solaire.
